# EUROPEAN PATENT SPECIFICATION

(11) **EP 2 304 016 B1**
(45) Date of publication and mention of the grant of the patent: **27.06.2012**
(21) Application number: 09790141.7
(22) Date of filing: 08.07.2009
(51) Int. Cl.: C12M 1/00, B01L 3/00

(54) **Micro-fluidic cell manipulation and holding device**
Mikrofludische Manipulations- und Haltevorrichtung für eine Zelle
Dispositif microfluidique de manipulation et de conservation de cellules

(30) Priority: 16.07.2008 US 135049
(43) Date of publication of application: 06.04.2011
(73) Proprietor: Cook Medical Technologies LLC, Bloomington, IN 47404 (US); William A. Cook Australia Pty. Ltd., Eight Mile Plains Brisbane, QLD 4113 (AU)
(72) Inventor: JUNGER, Michael, Carl, Brookfield Queensland 4069 (AU); PATEN, Katie, L., Marsden Queensland 4132 (AU); GILIAM, Kim, John, Wakerley Brisbane 4154 (AU); O'BRIEN, David Sean, Queensland 4165 (AU); THOMPSON, Jeremy, Grange, SA 5022 (AU)
(74) Representative: Garratt, Peter Douglas
(86) International application number: PCT/US2009/049895
(87) International publication number: WO 2010/008977

(56) References cited:
- WO-A-2006/097749
- US-A1- 2007 264 705

## Description

### BACKGROUND

Technical Field

The present invention relates to a device for manipulating or holding cells, such as culturing cells or tissues. More specifically, the invention relates to a micro-fluidic device for manipulating or holding cells or tissues.

Background of the Invention

Micro-fluidics is the technology used to design, model, manufacture and mass-produce micro-systems that handle fluids - gases, vapours or liquids in volumes that can be as small as micro, nano or pico liters. Active and passive microstructures control the flow and mixing of the fluids to produce physical, chemical, biochemical and microbiological reactions in a rapid, cost-effective manner. Micro-fluidics have a range of applications, including the handling and culture of cells and the automation of highly manual laboratory processes, such as *in vitro* fertilization (IVF), onto a single substrate.

When handling an embryonic cell, it is desirable to be able to hold it at a certain orientation or to manipulate it into a selected orientation for morphological examination and then hold it at that orientation for observation and treatment. This is particularly the case during intracytoplasmic sperm injection (ICSI), where the orientation of the polar body of the oocyte to the injection site is very important.

Document US-A-2007/0264705 describes a micro-fluidic manipulation device comprising wells dimensioned to receive a cell in order to allow observation of the cell. The position of the cell cannot be controlled in this device.

### SUMMARY

The invention will be discussed in relation to its application for cell manipulation and treatment but its application is not so limited and can also relate to cell and tissue culture more generally. The invention has particular application to mammalian cell culture including mammalian cell replication and/or reproduction. In one application, the invention is used for culturing embryos for IVF.

The invention will be discussed in relation to embryonic cells but is not so limited and may apply to other forms of cells or similar organisms.

The present invention seeks to provide a device which enables manipulation and treatment of a cell at a micro-fluidic volume or level or to at least provide the practitioner in the field with a useful device.

In one embodiment, the invention relates to a micro-fluidic manipulation device. The device includes a body, at least one well in the formed in the body, the well including a base and a side wall, the well being dimensioned to receive a cell selected from the group consisting of an oocyte, embryonic cell or somatic cell. The device also includes at least one base fluid port in the base of the well and at least one side fluid port in the side wall, each of the at least one base fluid port and the at least one side fluid port including a cross sectional area dimensioned to prevent the cell passing therethrough and being connected to manipulation flow ducts in the body. Selected flow of fluid through the base fluid port in use assists in holding the cell; and selected flow of fluid through the base fluid port and the at least one side port in use assists in rotating the cell in the well.

Preferably, the device includes two pairs of diametrically opposed side fluid ports in the side wall.

Preferably, each of the at least one base fluid port and the at least one side fluid port comprises a non-circular cross sectional area, whereby to prevent a seal being formed when the cell is retained thereon by the selected fluid flow. The non-circular cross sectional area can be selected from shapes, such as star shape, elliptical shape, hemi-spherical, hemi-elliptical, square shape, rectangular shape, and other suitable shape.

The base fluid port may be used to supply to or withdraw fluid from the well. For instance, it would be possible to draw fluid from the base port in order to hold the oocyte, embryonic cell or somatic cell while exchanging cell media through the port.

In another embodiment, the well includes a retention section and a loading section, the loading section being above and larger than the retention section, and the at least one side fluid port opening into a lower region of the loading section. The loading section may include a loading channel extending thereto. Alternatively, the loading region may be accessed from directly over the top of the loading port. Each of the at least one base fluid port and the at least one side fluid port may be supplied by micro-fluidic flow ducts in the body.

There can be further included at least one cell media withdrawal duct extending from the well. By having more than one separate cell media exchange duct, various media may be switched between/flushed. The media withdrawal duct or ducts extending from the well can comprise a cross sectional area dimensioned to prevent the cell passing there through.

Alternatively, there can be a plurality of cell media withdrawal ducts extending from the well, where each media withdrawal duct comprises a cross sectional area dimensioned to prevent the cell passing therethrough.

The media withdrawal duct may be at an upper end of the loading cell whereby to provide minimal disturbance to flow of fluid in the well.

Accordingly, the invention relates to a micro-fluidic device which can hold a cell, such as an oocyte embryonic cell, or a somatic cell, and provides fluid flow through respective ports so that the cell can be manipulated, such as being rotated to a selected position or the cell can be held in place for any necessary treatment.

### BRIEF DESCRIPTION OF THE DRAWINGS

This then generally describes the invention but to assist with understanding reference will now be made to the accompanying drawings, which show certain selected embodiments of the invention.

In the drawings:

Figure 1 shows a plan view of a first embodiment of cell manipulation and holding device according to the present invention;

Figure 2 shows a part cross sectional view of the embodiment of cell manipulation and holding device along the line 2 - 2' in Figure 1;

Figure 3 shows a part cross sectional view of the embodiment of cell manipulation and holding device along the line 3 - 3' in Figure 1;

Figure 4 shows a part cross sectional view of the embodiment of cell manipulation and holding device along the line 4 -4' in Figure 1;

Figure 5 shows a detailed view of one embodiment of the well of a cell manipulation and holding device according to the present invention;

Figure 6 shows a detailed view of an alternative embodiment of the well of a cell manipulation and holding device according to the present invention;

Figures 7A to 7D show still further embodiments of the well of a cell manipulation and holding device according to the present invention;

Figure 8 shows an alternative embodiment of cell manipulation and holding device according to the present invention;

Figure 9 shows detail of an alternative embodiment of well particularly showing an arrangement of diffuse outlet ports;

Figure 10 shows an alternative embodiment of cell manipulation and holding device according to the present invention; and

Figure 11 shows detail of the embodiment of Figure 10 in cross section along the line 11 - 11' in Figure 10.

### DETAILED DESCRIPTION

The following detailed description and appended drawings describe and illustrate various exemplary embodiments of the invention. The description and drawings serve to enable one skilled in the art to make and use the invention.

Definitions:

It should be understood that the terms "a" and "an" as used above and elsewhere herein refer to "one or more" of the enumerated components. For example, "a" cell refers to one cell or a mixture comprising two or more cells.

As used herein the terms "comprise(s)," "include(s)," "having," "has," "contain(s)," and variants thereof, are intended to be open-ended transitional phrases, terms, or words that do not preclude the possibility of additional acts or structure.

The term "oocyte" refers to a cell from which an egg or ovum develops by meiosis; a female gametocyte.

The terms "embryonic cell," "embryonic stem cell," or "pluripotent stem cell," may be used interchangeably and refer to one of the cells that are self-replicating, are derived from embryos, such as human embyos or human fetal tissue, and are known to develop into cells and tissues of the three primary germ layers. Although human pluripotent stem cells may be derived from embryos or fetal tissue, such stem cells are not themselves embryos. (See the National Institutes of Health Guidelines for Research Using Human Pluripotent Stem Cells.) "Self-replicating" means the cell can divide and form cells indistinguishable from it. The "three primary germ layers" -- called the ectoderm, mesoderm, and endoderm -- are the primary layers of cells in the embryo from which all tissues and organs develop.

The term "somatic cell" refers to any cell forming the body of an organism, as opposed to germline cells. Somatic cells may be of mammalian and non-mammalian origin. For example, in mammals, germline cells (also known as "gametes") are the spermatozoa and ova which fuse during fertilization to produce a cell called a zygote, from which the entire mammalian embryo develops. Every other cell type in the mammalian body-apart from the sperm and ova, the cells from which they are made (gametocytes) and undifferentiated stem cells—is a somatic cell: internal organs, skin, bones, blood, and connective tissue are all made up of somatic cells.

The present invention relates to a cell manipulation and holding device and methods of use and manufacture thereof. The cells to be manipulated or held include but are not limited to an oocyte, embryonic cell or somatic cell.

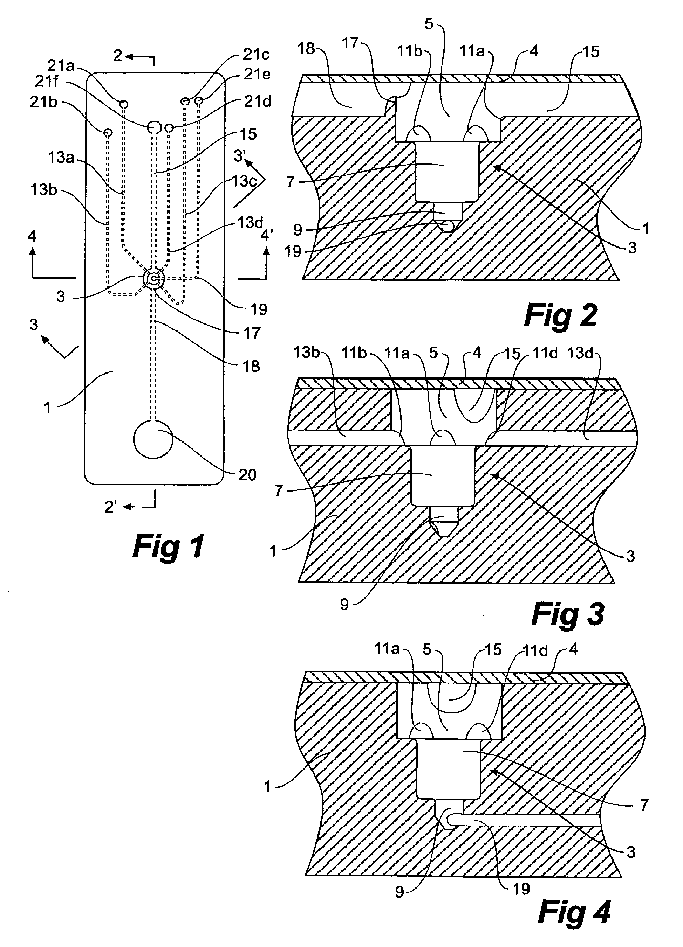

Referring to Figures 1 to 4, in one embodiment, a cell manipulation and holding device **1** according to the present invention has a substantially rectangular body. The body can be made from glass or plastics material and may be assembled from a plurality of layers to facilitate formation of the features as discussed below. The body may have coatings or the like as is well known in the art. The body **1** has a cover plate 4, which can be removed to allow access to the well.

The device **1** has a well **3**, which as can be seen in Figure 2. The well has a loading section **5** and a retention section **7** and a base fluid port **9**. The retention section **7** is below the loading section **5** and the base fluid port **9** is below the retention section **7**. A number of control ports **11a** to **11d** extend into the loading section towards the lower end of the loading section. The control ports are spaced evenly around the loading section. In this embodiment, the control ports are arranged in opposite pairs, but in an alternative embodiment there may be three ports at 120 degrees to each other or any other convenient number. The control ports are of a lesser diameter than the expected diameter of a cell to be handled in the well, such an oocyte, embryonic cell, or somatic cell, to prevent a cell from entering the control port.

A series of supply ducts **13a** to **13d** extends to each control port **11a** to **11d**, respectively. A loading duct **15** extends to the loading section and a diffuse outlet port **17** and duct **18** extends away from the loading port. The diffuse outlet port **17** is of lesser diameter than the loading port to prevent a cell from moving from the loading chamber into the diffuse outlet port **17**. There may be more than one diffuse outlet port so that flow of media out of the well via the diffuse outlet port or ports **17** does not affect significantly the position of a cell in the well. A media withdrawal duct **18** extends to a waste well **20** from which the excess media can be withdrawn.

A manipulation duct **19** extends to the base fluid port **9**. Each of the supply ducts **13a** to **13d**, the loading duct **15**, and the manipulation duct **19** extend to suitable connection points **21a** to **21f** for selective fluid supply to the device. The connection point **21f** is larger than the other ports to allow for placement of a cell for transport along the loading duct **15** to the loading section **5** of the well **3**.

In use, a cell is placed into the connection point **21** and carried on a fluid flow along the loading duct **15** to the loading section **5** of the well **3**. The cell is retained in the retention section **7** for observation and the like. It is desirable to be able to rotate the cell for observation of the cell and hence a low fluid flow is provided through the manipulation duct **19** to lift the cell slightly into the loading section. The flow through one or more of the control ports **11a** to **11d** is used to rotate the cell as desired. Once a cell has been positioned at a selected orientation, the flow through the manipulation duct **19** is stopped and the cell is allowed to move into the retention section under a low positive pressure from the control ports. At this stage operations can be performed on the cell, such as injection if a suitable aperture (not shown) is provided in the cover plate **4** or the cover plate **4** can be removed to provide access to the well.

Alternatively, once a cell has been positioned at a selected orientation the flow through the manipulation duct **19** can be stopped and fluid withdrawn through the manipulation duct **19** so that the cell is caused to move back into the retention section and held against the base fluid port **9** by suction through the manipulation duct **19**.

To prevent a cell held against the base fluid port, either by flow at low positive pressure from the control ports or by withdrawal of fluid from the base fluid port, from causing the cell to jam into and block the base fluid port 9 the base fluid port **9** may be formed in a shape other than circular.

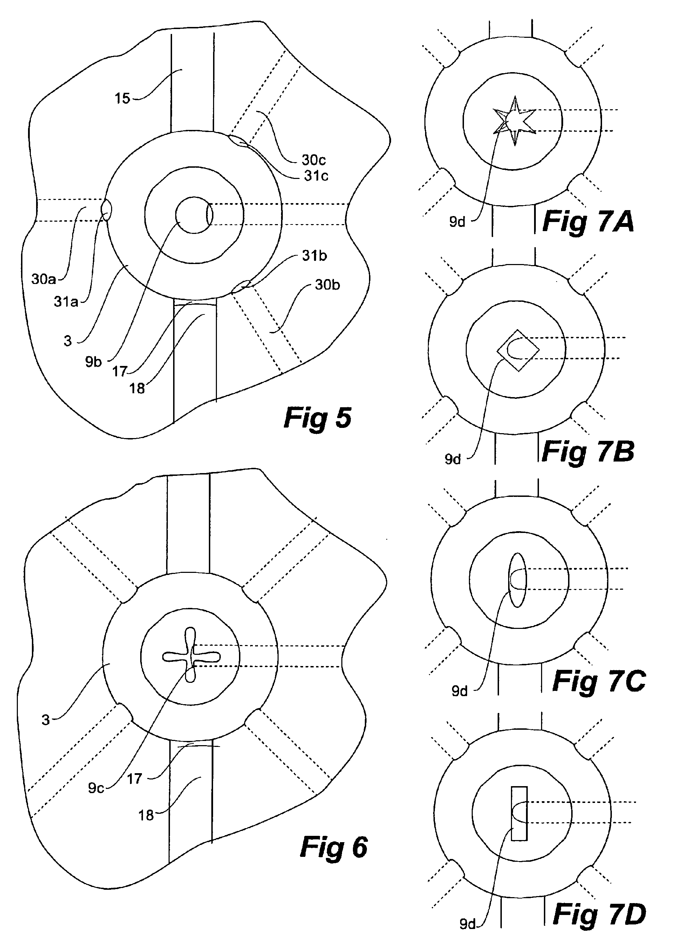

Figure 5 shows detail of one embodiment of the well **3** in plan view with the base fluid port **9b** being circular. Also, in this embodiment there are three supply ducts **30a** to **30c** and three control ports **11a** to **11c**.

Figure 6 shows details of another embodiment of the well **3** in plan view with the base fluid port **9c** being cloverleaf shaped.

Figures 7A to 7D show further alternative shapes of base fluid ports. In Figure 7A the base fluid port **9d** is of a star shape. In Figure 7B the base fluid port **9e** is of a diamond shape. In Figure 7C the base fluid port **9f** is of an elliptical shape. In Figure 7A the base fluid port **9g** is of a rectangular shape.

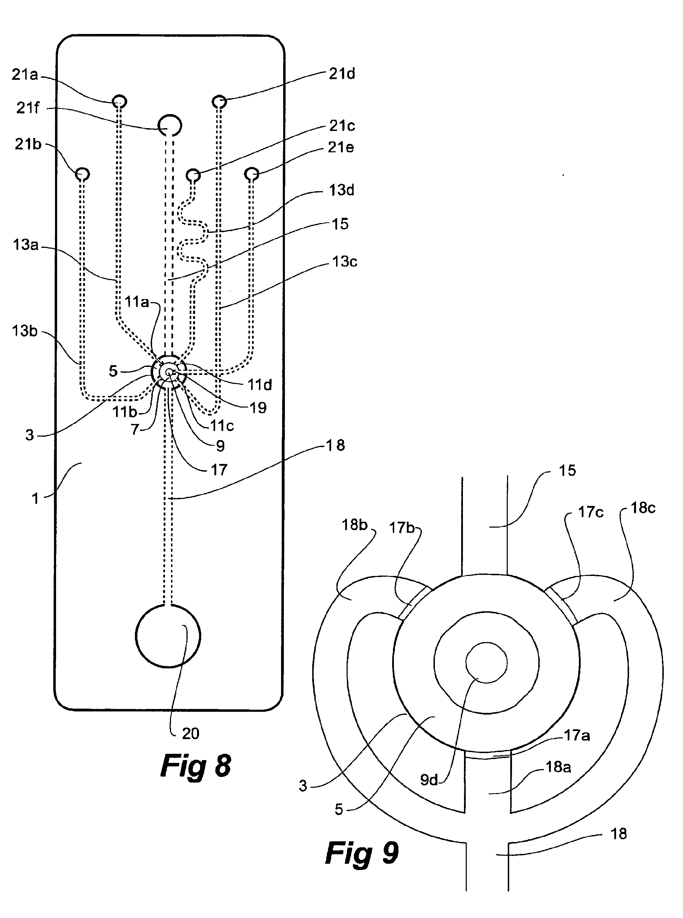

Figure 8 shows an alternative embodiment of the cell manipulation and holding device according to the present invention. In this embodiment, the same reference numerals have been used for corresponding items as those shown in Figures 1 to 4.

In Figure 8 the device **1** has a well **3**. A number of control ports **11a** to **11d** extend into a loading section **5** towards the lower end of the loading section **5**. The control ports are spaced evenly around the loading section. The control ports are of a lesser diameter than the expected diameter of a cell to be handled in the well to prevent a cell from entering a control port. A series of supply ducts **13a** to **13d** extends to each control port **11a** to **11d**, respectively. A loading duct **15** extends to the loading section **5** and a diffused outlet duct **18** extends away from a diffused outlet port **17** on the loading section. The diffused outlet port **17** is of lesser diameter than the diameter of the loading port to prevent a cell from moving from the loading chamber into the diffused outlet port **17**. There may be more than one diffused outlet port so that flow of media out of the well via the diffused outlet port or ports **17** does not affect significantly the position of a cell in the well. The media withdrawal duct **18** extends to a waste well **20** from which excess media can be withdrawn.

A manipulation duct **19** extends to the base fluid port **9**. Each of the supply ducts **13a** to **13d**, the loading duct **15**, and the manipulation duct **19** extend from suitable connection points **21a** to **21f** for fluid supply to the device. The connection point **21f** is larger than the other ports to allow for placement of a cell for transport along the loading duct **15** to the loading section **5** of the well **3**.

It is advantageous to have all of the control ducts to be the same length from the respective supply ports **21a** to **21d** to the control ports **11a** to **11d.** This ensures that an equal amount of flow in one of these ducts caused the same expected action in the well. Hence the duct **13c** is convoluted to make it the same overall length as the others. The respective supply ports **21a** to **21d** are spaced apart on the body **1** to allow for convenient connection to a suitable fluid supply for each supply port.

Figure 9 shows details of an alternative embodiment of well particularly showing an arrangement of diffuse outlet ports. In this embodiment, the well **3** has a loading duct **15** and three diffuse outlet ports **17a** to **17c** with ducts **18a** extending to the waste duct **18**.

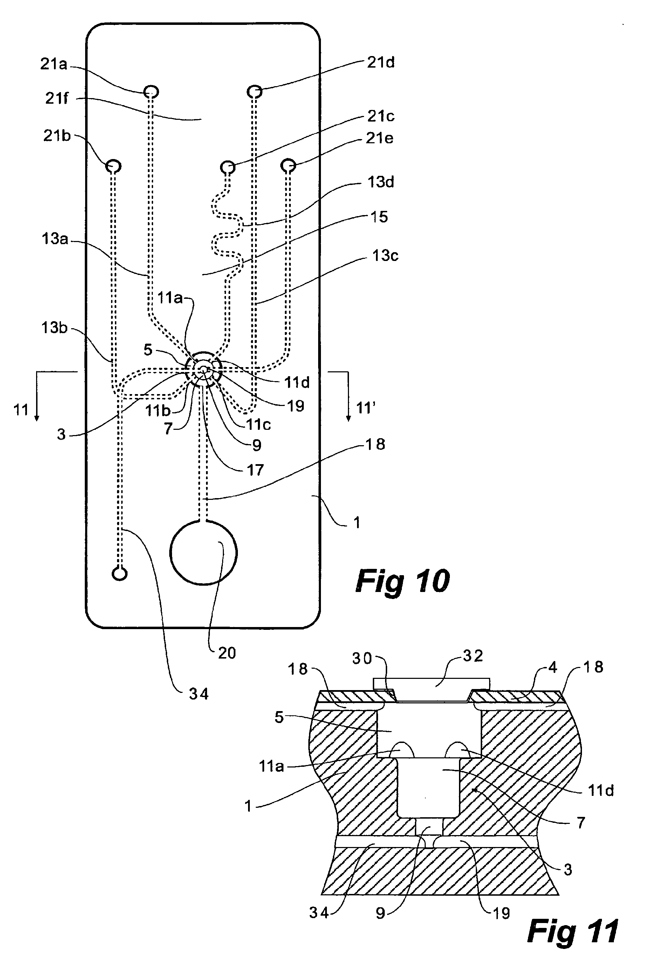

Figure 10 shows an alternative embodiment of the cell manipulation and holding device according to the present invention. Figure 11 shows details of the embodiment of Figure 10 in cross section along the line 11 - 11' in Figure 10. In this embodiment, the same reference numerals have been used for corresponding items as those shown in Figures 1 to 4.

In this embodiment, the cell manipulation and holding device according to this embodiment has a substantially rectangular body **1**. The body is illustrated as rectangular but can be any other convenient shape. The body can be made from glass or plastics material and may be assembled from a plurality of layers to facilitate formation of the features as discussed herein. The body may have coatings or the like as is well known in the art. The body **1** can have a cover plate **4**, which can be removed to allow access to the well.

The device **1** has a well **3**, which as can be seen in Figure **11**, has a loading section **5** and a retention section **7** and a base fluid port **9.** The retention section **7** is below the loading section **5** and the base fluid port **9** is below the retention section **7**. A number of control ports **11a** to **11d** extend into the loading section towards the lower end of the loading section. The control ports are spaced evenly around the loading section. In this embodiment, the control ports are arranged in opposite pairs, but in an alternative embodiment there may be three ports at 120 degrees to each other, or any other convenient number. The control ports are of a lesser diameter than the expected diameter of a cell to be handled in the well to prevent a cell from entering a control port.

A series of supply ducts **13a** to **13d** extends to each control port **11a** to **11d**, respectively. A loading aperture **30** is directly above the loading section, and a diffuse outlet port **17** and duct **18** extend away from the loading port. A removable lid **32** in the loading aperture **30** can be removed to allow access to the well. The diffuse outlet port **17** is of lesser diameter than the expected diameter of a cell which may be placed into the well to prevent a cell from moving from the loading chamber into the diffuse outlet port **17**. There may be more than one diffuse outlet port so that flow of media out of the well via the diffuse outlet port or ports **17** does not affect significantly the position of a cell in the well. A media withdrawal duct **18** extends to a waste well **20** from which excess media can be withdrawn.

The base fluid port **9** has two ducts extending to it. A first duct is a supply duct **19** and a second is a withdrawal duct **34**. As described above with reference to other embodiments, the base port 9 preferably has a non-circular cross section to enable a cell to be held against the port but still enabling a small flow of media to be drawn into the base fluid port and out of the withdrawal duct **34**. This arrangement may allow for a cell be held in a selected orientation for treatment, as discussed above.

## Claims

1. A micro-fluidic manipulation device comprising
a body;
at least one well in the body, the well comprising a base and a side wall, the well being dimensioned to receive a cell selected from the group consisting of an oocyte, embryonic cell or somatic cell;
at least one base fluid port in the base of the well supplied by a micro-fluidic manipulation flow duct in the body and;
at least one side fluid port in the side wall supplied by a micro-fluidic supply duct in the body,
wherein each of the at least one base fluid port and the at least one side fluid port comprising a cross sectional area dimensioned to prevent the cell passing therethrough,
wherein selected flow of fluid through the base fluid port in use assists in holding the cell, and
wherein selected flow of fluid through the base fluid port and the at least one side fluid port in use assists in rotating the cell in the well.

2. The micro-fluidic manipulation device of claim 1, comprising two pairs of diametrically opposed side fluid ports in the side wall.

3. The micro-fluidic manipulation device of claim 1, wherein each of the at least one base fluid port and the at least one side fluid port comprises a non-circular cross sectional area to prevent a seal being formed when a cell is retained thereon by the selected fluid flow.

4. The micro-fluidic manipulation device of claim 3, wherein the non- circular cross sectional area is of star shape, elliptical shape, hemispherical, hemi-elliptical, square shape, or rectangular shape.

5. The micro-fluidic manipulation device of claim 3, wherein the non- circular cross sectional area comprises a filter membrane.

6. The micro-fluidic manipulation device of claim 1, wherein the well comprises
a retention section; and
a loading section, the loading section being disposed above and larger than the retention section, the loading section comprising a loading channel extending thereto and the at least one side fluid port opening into a lower region of the loading section.

7. The micro-fluidic manipulation device of claim 1, further comprising at least one media withdrawal duct extending from the well.

8. The micro-fluidic manipulation device of claim 7, wherein the media withdrawal duct comprises a cross sectional area dimensioned to prevent a cell passing into the duct.

9. The micro-fluidic manipulation device of claim 1, further comprising a plurality of media withdrawal ducts extending from the well, wherein each media withdrawal duct comprises a cross sectional area dimensioned to prevent a cell passing therethrough.

## Patentansprüche

1. Mikrofluidische Manipulationsvorrichtung, umfassend:
einen Körper;
mindestens eine Vertiefung im Körper, wobei die Vertiefung eine Basis und eine Seitenwand umfasst, wobei die Vertiefung zum Aufnehmen einer Zelle dimensioniert ist, die aus der Gruppe bestehend aus einer Eizelle, embryonalen Zelle oder Körperzelle ausgewählt ist;
mindestens einen Basisfluidport in der Basis der Vertiefung, der durch einen mikrofluidischen Manipulationsströmungskanal im Körper versorgt ist; und
mindestens einen Seitenfluidport in der Seitenwand, der durch einen mikrofluidischen Zufuhrkanal im Körper versorgt ist,
wobei jeder des mindestens einen Basisfluidports und des mindestens einen Seitenfluidports eine Querschnittsfläche umfasst, die so dimensioniert ist, dass sie den Durchgang der Zelle durch dieselbe verhindert,
wobei die gewählte Strömung des Fluids durch den Basisfluidport, der benutzt wird, dabei hilft, die Zelle zu halten, und
wobei die gewählte Strömung des Fluids durch den Basisfluidport und den mindestens einen Seitenfluidport, der benutzt wird, beim Drehen der Zelle in der Vertiefung hilft.

2. Mikrofluidische Manipulationsvorrichtung nach Anspruch 1, die zwei Paar von diametral entgegengesetzten Seitenfluidports in der Seitenwand umfasst.

3. Mikrofluidische Manipulationsvorrichtung nach Anspruch 1, wobei jeder von dem mindestens einen Basisfluidport und dem mindestens einen Seitenfluidport eine nicht kreisförmige Querschnittsfläche umfasst, um zu verhindern, dass sich ein Verschluss bildet, wenn eine Zelle durch die gewählte Fluidströmung darauf zurückgehalten wird.

4. Mikrofluidische Manipulationsvorrichtung nach Anspruch 3, wobei die nicht kreisförmige Querschnittsfläche eine Sternform, elliptische Form, halbkugelige, halb-elliptische, quadratische oder rechteckige Form besitzt.

5. Mikrofluidische Manipulationsvorrichtung nach Anspruch 3, wobei die nicht kreisförmige Querschnittsfläche eine Filtermembran umfasst.

6. Mikrofluidische Manipulationsvorrichtung nach Anspruch 1, wobei die Vertiefung Folgendes umfasst:
einen Rückhalteabschnitt; und
einen Ladeabschnitt, wobei der Ladeabschnitt oberhalb des Rückhalteabschnitts liegt und größer als derselbe ist, wobei der Ladeabschnitt einen Beladungskanal, der sich dorthin erstreckt, und den mindestens einen Seitenfluidport umfasst, der sich in einen tieferen Bereich des Ladeabschnitts öffnet.

7. Mikrofluidische Manipulationsvorrichtung nach Anspruch 1, die ferner mindestens einen Medienaustrittskanal umfasst, der sich von der Vertiefung aus erstreckt.

8. Mikrofluidische Manipulationsvorrichtung nach Anspruch 7, wobei der Medienaustrittskanal eine Querschnittsfläche umfasst, die so dimensioniert ist, dass sie den Durchgang einer Zelle in den Kanal verhindert.

9. Mikrofluidische Manipulationsvorrichtung nach Anspruch 1, die ferner mehrere Medienaustrittskanäle umfasst, die sich von der Vertiefung aus erstrecken, wobei jeder Medienaustrittskanal eine Querschnittsfläche umfasst, die so dimensioniert ist, dass sie den Durchgang einer Zelle durch dieselbe verhindert.

## Revendications

1. Dispositif microfluidique de manipulation, comprenant:
un corps;
au moins un puits dans le corps, le puits comprenant une base et une paroi latérale, le puits étant dimensionné de manière à recevoir une cellule sélectionnée dans le groupe comprenant un ovocyte, une cellule embryonnaire ou une cellule somatique;
au moins un port de fluide de base dans la base du puits alimenté par un conduit d'écoulement de manipulation microfluidique dans le corps; et
au moins un port de fluide latéral dans la paroi latérale alimenté par un conduit d'alimentation microfluidique dans le corps,
dans lequel chacun dudit moins un port de fluide de base et dudit au moins un port de fluide latéral présente une surface de section transversale qui est dimensionnée de manière à empêcher la cellule de passer à travers celui-ci,
dans lequel, lors de l'utilisation, un écoulement de fluide sélectionné à travers le port de base aide à conserver la cellule, et
dans lequel, lors de l'utilisation, un écoulement de fluide sélectionné à travers le port de fluide de base et ledit au moins un port de fluide latéral aide à faire tourner la cellule dans le puits.

2. Dispositif microfluidique de manipulation selon la revendication 1, comprenant deux paires de ports de fluide latéraux diamétralement opposés dans la paroi latérale.

3. Dispositif microfluidique de manipulation selon la revendication 1, dans lequel chacun desdits au moins un port de fluide de base et desdits au moins un port de fluide latéral présente une surface de section transversale non circulaire destinée à empêcher la formation d'un joint lorsqu'une cellule est retenue sur celui-ci par l'écoulement de fluide sélectionné.

4. Dispositif microfluidique de manipulation selon la revendication 3, dans lequel la surface de section transversale non circulaire est en forme d'étoile, de forme elliptique, de forme hémisphérique, de forme hémi-elliptique, de forme carrée ou de forme rectangulaire.

5. Dispositif microfluidique de manipulation selon la revendication 3, dans lequel la surface de section transversale non circulaire comprend une membrane de filtrage.

6. Dispositif microfluidique de manipulation selon la revendication 1, dans lequel le puits comprend:
une section de rétention; et
une section de chargement, la section de chargement étant disposée au-dessus de la section de rétention et étant plus grande que celle-ci, la section de chargement comprenant un canal de chargement qui s'étend vers celle-ci, et ledit au moins un port de fluide latéral s'ouvrant dans une région inférieure de la section de chargement.

7. Dispositif microfluidique de manipulation selon la revendication 1, comprenant en outre au moins un conduit de prélèvement de milieu qui s'étend à partir du puits.

8. Dispositif microfluidique de manipulation selon la revendication 7, dans lequel le conduit de prélèvement de milieu présente une surface de section transversale qui est dimensionnée de manière à empêcher une cellule de passer dans le conduit.

9. Dispositif microfluidique de manipulation selon la revendication 1, comprenant en outre une pluralité de conduits de prélèvement de milieu qui s'étendent à partir du puits, dans lequel chaque conduit de prélèvement de milieu présente une surface de section transversale qui est dimensionnée de manière à empêcher une cellule de passer à travers celui-ci.
